(19) Europäisches Patentamt
European Patent Office
Office européen des brevets

(11) EP 1 819 331 B1

(12) **EUROPEAN PATENT SPECIFICATION**

(45) Date of publication and mention of the grant of the patent:
**14.10.2009 Bulletin 2009/42**

(21) Application number: **05852316.8**

(22) Date of filing: **28.11.2005**

(51) Int Cl.:
***A61K 31/426*** *(2006.01)* ***A61K 31/519*** *(2006.01)*
***A61K 31/436*** *(2006.01)* ***A61P 35/00*** *(2006.01)*

(86) International application number:
**PCT/US2005/042975**

(87) International publication number:
**WO 2006/060331 (08.06.2006 Gazette 2006/23)**

(54) **COMBINATIONS COMPRISING EPOTHILONES AND PROTEIN TYROSINE KINASE INHIBITORS AND PHARMACEUTICAL USES THEREOF**

KOMBINATIONEN MIT EPOTHILONEN UND PROTEIN-TYROSIN-KINASEHEMMERN SOWIE PHARAMAZEUTISCHE VERWENDUNG DAFÜR

PRODUITS COMPOSES CONTENANT DES EPOTHILONES ET DES INHIBITEURS DES PROTEINES TYROSINE KINASES, ET LEURS UTILISATIONS PHARMACEUTIQUES

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**HR**

(30) Priority: **30.11.2004 US 631837 P**

(43) Date of publication of application:
**22.08.2007 Bulletin 2007/34**

(73) Proprietors:
- **Novartis AG**
  **4056 Basel (CH)**
- **NOVARTIS-PHARMA GMBH**
  **1230 Vienna (AT)**
  Designated Contracting States:
  **AT**

(72) Inventors:
- **HUANG, Jerry, Min-Jian**
  **Florham Park, New Jersey 07932 (US)**
- **JOHRI, Anandhi, Ranganathan**
  **Short Hills, New Jersey 07078 (US)**
- **LINNARTZ, Ronald, Richard**
  **Andover, New Jersey 07821 (US)**
- **MCSHEEHY, Paul, M., J.**
  **79540 Loerrach-Stetten (DE)**

(74) Representative: **Roth, Peter Richard**
**Novartis AG**
**Corporate Intellectual Property**
**4002 Basel (CH)**

(56) References cited:
**WO-A-02/067941** **WO-A-03/013541**
**WO-A-03/035047** **WO-A-03/103712**
**WO-A-20/04004644** **FR-A- 2 775 187**

- **JOENSUU H ET AL: "TYROSINE KINASE INHIBITOR IMATINIB (STI571) AS AN ANTICANCER AGENT FOR SOLID TUMOURS" ANNALS OF MEDICINE, FINNISH MEDICAL SOCIETY DUODECIM, HELSINKI, FI, vol. 33, no. 7, 2001, pages 451-455, XP001131999 ISSN: 0785-3890**
- **SHAH N P ET AL: "RECENT SUCCESS WITH THE TYROSINE KINASE INHIBITOR STI-571 - LESSONS FOR TARGETED THERAPY OF CANCER" CURRENT OPINION IN INVESTIGATIONAL DRUGS, PHARMAPRESS, US, vol. 2, no. 3, March 2001 (2001-03), pages 422-423, XP009017073 ISSN: 1472-4472**
- **M FAILLY ET AL.: "Combination of subletal concentrations of epidermal growth factor receptor inhibitor and microtubule stabilizer induces apoptosis of glioblastoma cells" MOLECULAR CANCER THERAPY, vol. 6, no. 2, - 2007 pages 773-781,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Remarks:

The file contains technical information submitted after the application was filed and not included in this specification

## Description

The invention relates to a combination which comprises:

(a) an epothilone derivative of formula (I')

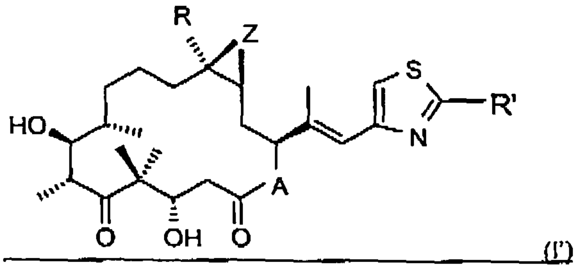

(I')

wherein A represents O or $NR_N$. wherein $R_N$ is hydrogen or lower alkyl, R is hydrogen or lower alkyl, R' is methyl, and Z is O or a bond, in free form or in the form of a pharmaceutically acceptable salt; and
(b) a protein tyrosine kinase inhibitor {6-[4-(ethyl-piperazin-1-ylmethyl)-phenyl]-7*H*-pyrrolo[2,3-*d*]pyrimidin-4-yl}-(1-[phenyl-ethyl)-amine; and optionally
(c) a derivative of rapamycin; for simultaneous, separate or sequential use, in particular, for the delay of progression or treatment of a proliferative disease, especially cancer.

The invention also relates to a pharmaceutical composition comprising such a combination and optionally at least one pharmaceutically acceptable carrier. The invention also relates to the use of such a combination for the preparation of a medicament for the delay of progression or treatment of a proliferative disease. The invention also relates to a commercial package or product comprising such a combination as a combined preparation for simultaneous, separate or sequential use.

## Background of the Invention

The epothilones represent a class of microtubule stabilizing cytotoxic agents. See Gerth et al., J Antibiot, Vol. 49, pp. 560-563 (1966); or Hoefle et al., DE 41 38 042. They are 16-member macrolides containing seven, chiral centers and may also be characterized by various functionalities. For example, they may include other ring systems, such as an epoxide and/or a thiazole ring, They may have two free, derivatizable hydroxyl groups and the macrolide itself may comprise an ester linkage.

Cytotoxic agents are well-known for the treatment of tumors. The anti-tumor activity of many of these compounds relies on the inhibition of cell proliferation and consequent induction of apoptosis and cell death. The majority of cytotoxic agents exert their effects through interference of DNA and/or RNA syntheses. However, for certain cytotoxic agents, e.g., members of the taxane family, e.g., paclitaxel; and the epothilones, their activity is reliant on their interference with microtubule dynamics. Microtubules are an important and attractive target for development of novel anti-cancer formulations.

Protein tyrosine kinase inhibitors are widely used to inhibit protein tyrosine kinase activity in a variety of both benign and malignant diseases. Protein tyrosine kinase receptors play a key role in signal transmission in a large number of mammalian cells, including human cells, especially epithelial cells, cells of the immune system and cells of the central and peripheral nervous system. Most importantly, overexpression of these receptors has been observed in substantial fractions of many human tumors.

## Summary of the Invention

It has now been found that surprisingly the administration of an epothilone with a protein tyrosine kinase inhibitor and optionally the addition of a derivative of rapamycin is useful for the treatment of a proliferative disease, especially cancer.

Accordingly, the present invention provides a combination, such as a combined preparation or a pharmaceutical composition, which comprises:

(a) an epothilone derivative of formula (I');

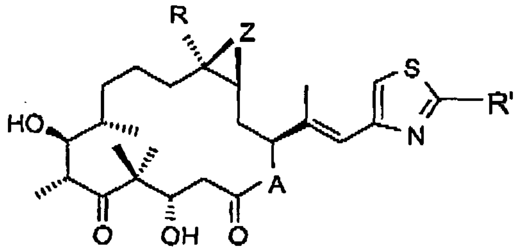

(I')

wherein A represents O or $NR_N$, wherein $R_N$ is hydrogen or lower alkyl, R is hydrogen or lower alkyl, R' is methyl, and Z is O or a bond, in free form or In the form of a pharmaceutically acceptable salt; and

(b) a protein tyrosine kinase inhibitor {6-[4-(4-ethyl-piperazin-1-ylmethyl)-phenyl]-7H-pyrrolo[2,3-d]pyrimidin-4-yl}-(1-phenyl-ethyl)-amine; and optionally

(c) a derivative of rapamycin,

in which the active ingredients (a) and (b), and optionally (c), are present in each case in free form or in the form of a pharmaceutically acceptable salt, for simultaneous, concurrent, separate or sequential use in the treatment of a proliferative disease.

The term "a combined preparation", as used herein, defines especially a "kit of parts" in the sense that the combination partners (a) and (b), and optionally (c), as defined above, can be dosed independently or by use of different fixed combinations with distinguished amounts of the combination partners (a) and (b), and optionally (c), i.e., simultaneously or at different time points. The parts of the kit of parts can then, e.g., be administered simultaneously or chronologically staggered, that is at different time points and with equal or different time intervals for any part of the kit of parts. Very preferably, the time intervals are chosen such that the effect on the treated disease in the combined use of the parts is larger than the effect which would be obtained by use of only any one of the combination partners (a) and (b), and optionally (c). The ratio of the total amounts of the combination partner (a) to the combination partner (b), and optionally the addition of combination partner (c), to be administered in the combined preparation can be varied, e.g., in order to cope with the needs of a patient subpopulation to be treated or the needs of the single patient which different needs can be due to age, sex, body weight, etc. of the patients. Preferably there is at least one beneficial effect, e.g., a mutual enhancing of the effect of the combination partners (a) and (b), and optionally (c), in particular, a synergism, e.g., a more than additive effect, additional advantageous effects, less side effects, a combined therapeutical effect in a non-effective dosage of one or both of the combination partners (a) and (b), and optionally (c), and very preferably, a strong synergism of the combination partners (a) and (b), and optionally (c).

Further, the invention provides the use of an epothilone, for use in combination with a protein tyrosine kinase inhibitor and optionally a derivative of rapamycin, for treatment of a proliferative disease, especially a malignant disease, such as cancer.

In the alternative, the invention provides the use of a protein tyrosine kinase inhibitor, for use in combination with an epothilone and optionally a derivative of rapamycin for treatment of a proliferative disease, especially a malignant disease, such as cancer.

Diseases and conditions which may be treated in accordance with the present invention include breast cancer, ovarian cancer cancer of the colon and generally the gastrointestinal tract including gastric cancer; cervix cancer lung cancer, e.g., small-cell lung cancer and non-small-cell lung cancer; pancreas cancer; renal cancer; glioma; melanoma; head and neck cancer; bladder cancer; hepatocellular cancer; prostate cancer; and Kaposi's sarcoma.

Thus, in the present description, the terms "treatment" or "treat" refer to both prophylactic or preventative treatment, as well as curative or disease modifying treatment, including treatment of patients at risk of contracting the disease or suspected to have contracted the disease, as well as patient who are ill or have been diagnosed as suffering from a disease or medical condition.

## Detailed Description of the Present Invention

The epothilones of the present invention are derivatives of formula (I')

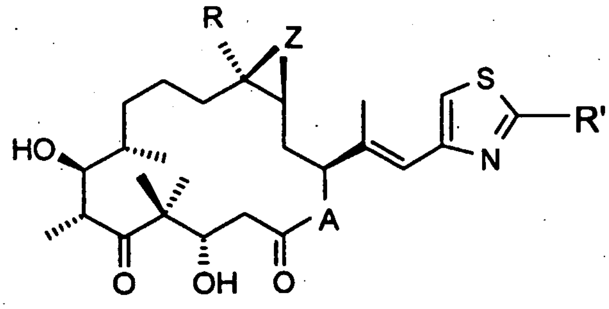

(I')

wherein A represents O or $NR_N$, wherein $R_N$ is hydrogen or lower alkyl, R is hydrogen or lower alkyl, R' is methyl, methoxy, ethoxy, amino, methylamino, dimethylamino, aminomethyl or methylthio, and Z is O or a bond, in free form or in the form of a pharmaceutically acceptable salt.

A more preferred embodiment are compounds of formula I

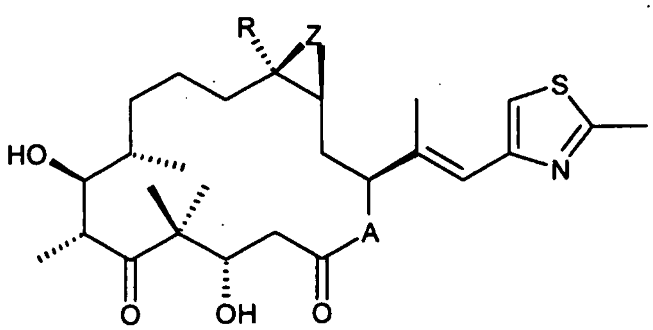

(I)

,

wherein
A represents O or $NR_N$, wherein $R_N$ is hydrogen or lower alkyl;
R is hydrogen or lower alkyl; and
Z is O or a bond.

Unless stated otherwise, in the present disclosure of the epothilones of formula (I) "lower" means not more than 7 carbon atoms, preferably not more than 4 carbon atoms.

A compound of formula (I),
wherein
A represents O;
R is hydrogen; and
Z is O,
is known as epothilone A.

A compound of formula (I),
wherein
A represents O;
R is methyl; and
Z is O,
is known as epothilone B.

A compound of formula (I),
wherein
A represents O;
R is hydrogen; and
Z is a bond,
is known as epothilone C.

A compound of formula (I),
wherein

A represents O;
R is methyl; and
Z is a bond,
is known as epothilone D.

Epothilone derivatives of formula (I),
wherein
A represents O or $NR_N$, wherein $R_N$ is hydrogen or lower alkyl;
R is hydrogen or lower alkyl; and
Z is O or a bond,
and methods for the preparation of such epothilone derivatives are, in particular, generically and specifically disclosed in the patents and patent applications WO 93/10121, U.S. Patent No. 6,194,181, WO 98/25929, WO 98/08849, WO 99/43653, WO and WO 00/31247 in each case, in particular, in the compound claims and the final products of the working examples. Comprised are likewise the corresponding stereoisomers, as well as the corresponding crystal modifications, e.g., solvates and polymorphs, which are disclosed therein. Epothilone derivatives of formula (I') or (I), especially epothilone B. can be administered as part of pharmaceutical compositions which are disclosed in WO 99/39694.

The protein tyrosine kinase inhibitors of the present invention are described in WO 03/013541, which is incorporated by reference, and are 7*H*-pyrrolo[2,3-*d*]pyrimidine derivatives of formula (II)

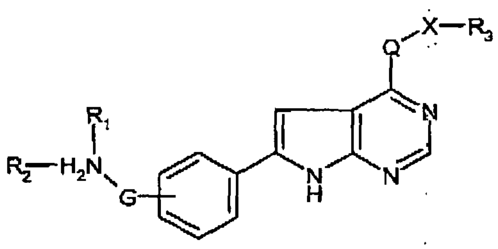

(II)

wherein
$R_1$ and $R_2$ are, each independently of the other hydrogen, unsubstituted or substituted alkyl or cycloalkyl, a heterocyclic radical bonded via a ring carbon atom or a radical of the formula $R_4$-Y-(C=Z)-,
wherein
$R_4$ is unsubstituted, mono- or di-substituted amino or a heterocyclic radical;
Y is either not present or lower alkyl; and
Z is oxygen, sulfur or imino, with the proviso that $R_1$ and $R_2$ are not both hydrogen, or
$R_1$ and $R_2$, together with the nitrogen atom to which they are attached, form a
heterocyclic radical; .
$R_3$ is a heterocyclic radical or an unsubstituted or substituted aromatic radical;
G is $C_1$-$C_7$alkylene, -C(=O)- or $C_1$-$C_6$-alkylene-C(=O)-, wherein the carbonyl group is attached to the $NR_1R_2$ moiety;
Q is -NH- or -O-, with the proviso that Q is -0- if G is -C(=O)- or $C_1$-$C_6$alkylene-C(=O)-; and
X is either not present or $C_1$-$C_7$alkylene, with the proviso that a heterocyclic radical $R_3$ is bonded via a ring carbon atom if X is not present;
or a salt of the compounds.

The general terms, used hereinbefore and hereinafter in regard to the protein tyrosine kinase inhibitors of formula (II), preferably have within the context of this disclosure the following meanings, unless otherwise indicated.

Where the plural form is used for compounds, salts and the like, this is taken to mean also a single compound, salt or the like.

Where compounds of formula (II) are mentioned which can form tautomers, it is meant to include also the tautomers of such compounds of formula (II). In particular, tautomerism occurs, e.g., for compounds of formula (II) which contain a 2-hydroxy-pyridyl radical. In such compounds the 2-hydroxy-pyridyl radical can also be present as pyrid-2(1*H*)-on-yl.

Asymmetric carbon atoms of a compound of formula (II) that are optionally present may exist in the (*R*), (*S*) or (*R*,*S*) configuration, preferably in the (*R*) or (*S*) configuration. Substituents at a double bond or a ring may be present in *cis*- (= Z-) or *trans* (= E-) form. The compounds may thus be present as mixtures of isomers or preferably as pure isomers.

Preferably alkyl of formula (II) contains up to 20 carbon atoms and is most preferably lower alkyl.

The prefix "lower" of formula (II( denotes a radical having up to and including a maximum of 7 carbon atoms, especially up to and including a maximum of 4 carbon atoms, the radicals in question being either unbranched or branched with single or multiple branching. Lower alkyl is, e.g., methyl, ethyl, *n*-propyl, isopropyl, *n*-butyl, isobutyl, *sec*-butyl,

*tert*-butyl, *n*-pentyl, isopentyl, neopentyl, *n*-hexyl or *n*-heptyl.

Alkyl $R_1$ and $R_2$ of formula (II), independently of each other, are preferably methyl, ethyl, isopropyl or *tert*-butyl, especially methyl or ethyl.

Lower alkyl Y of formula (II) is preferably methyl, ethyl or propyl.

Lower alkoxy of formula (II) is, e.g., ethoxy or methoxy, especially methoxy.

Substituted alkyl of formula (II) is preferably lower alkyl, as defined above, where one or more substituents, preferably one substituent may be present, such as, e.g., amino, *N*-lower alkylamino, *N*,*N*-di-lower alkylamino, *N*-lower alkanoylamino, *N*,*N*-di-lower alkanoylamino, hydroxy, lower alkoxy, lower alkanoyl, lower alkanoyloxy, cyano, nitro, carboxy, lower alkoxycarbonyl, carbamoyl, *N*-lower alkyl-carbamoyl, *N*,*N*-di-lower alkyl-carbamoyl, amidino, guanidino, ureido, mercapto, lower alkylthio, halogen or a heterocyclic radical.

Substituted alkyl $R_1$ and $R_2$ of formula (II) are, independently of each other, preferably hydroxy-lower alkyl, *N*,*N*-di-lower alkylamino-lower alkyl or morpholinyl-lower alkyl.

Preferably unsubstituted or substituted cycloalkyl $R_1$ or $R_2$ of formula (II) contains from 3 carbon atoms, up to 20 carbon atoms, and is especially unsubstituted or also substituted $C_3$-$C_6$cycloalkyl, wherein the substituents are selected from, e.g., unsubstituted or substituted lower alkyl, amino, *N*-lower alkylamino, *N*,*N*-di-lower alkylamino, *N*-lower alkanoylamino, *N*,*N*-di-lower alkanoylamino, hydroxy, lower alkoxy, lower alkanoyl, lower alkanoyloxy, cyano, nitro, carboxy, lower alkoxycarbonyl, carbamoyl, *N*-lower alkyl-carbamoyl, *N*,*N*-di-lower alkyl-carbamoyl, amidino, guanidino, ureido, mercapto, lower alkylthio, halogen or a heterocyclic radical.

Mono- or di-substituted amino of formula (II) is amino substituted by one or two radicals selected independently of one another from, e.g., unsubstituted or substituted lower alkyl.

Di-substituted amino $R_4$ of formula (II) is preferably *N*,*N*-di-lower alkylamino, especially *N*,*N*-dimethylamino or *N*,*N*-diethylamino.

A heterocyclic radical of formula (II) contains especially up to 20 carbon atoms and is preferably a saturated or unsaturated monocyclic radical having from 4 or 8 ring members and from 1-3 heteroatoms, which are preferably selected from nitrogen, oxygen and sulfur, or a bi- or tri-cyclic radical wherein, e.g., one or two carbocyclic radicals, such as, e.g., benzine radicals, are annellated (fused) to the mentioned monocyclic radical. If a heterocyclic radical contains a fused carbocyclic radical then the heterocyclic radical may also be attached to the rest of the molecule of formula (I) via a ring atom of the fused carbocyclic radical. The heterocyclic radical, including the fused carbocyclic radical(s) if present, is optionally substituted by one or more radicals, preferably by one or two radicals, such as, e.g., unsubstituted or substituted lower alkyl, amino, *N*-lower alkylamino, *N*,*N*-di-lower alkylamino, *N*-lower alkanoylamino, *N*,*N*-di-lower alkanoylamino, hydroxy, lower alkoxy, lower alkanoyl, lower alkanoyloxy, cyano, nitro, carboxy, lower alkoxycarbonyl, carbamoyl, *N*-lower alkyl-carbamoyl, *N*,*N*-di-lovirer alkyl-carbamoyl, amidino, guanidino, ureido, mercapto, lower alkylthio or halogen.

Most preferably, a heterocyclic radical of formula (II) is pyrrolidinyl, piperidyl, lower alkyl-piperazinyl, di-lower alkyl-piperazinyl, morpholinyl, tetrahydropyranyl, pyridyl, pyridyl substituted by hydroxy or lower alkoxy or benzodioxolyl, especially pyrrolidinyl, piperidyl, lower alkyl-piperazinyl, di-lower alkyl-piperazinyl or morpholinyl.

A heterocyclic radical $R_1$ or $R_2$ of formula (II) is as defined above for a heterocyclic radical with the proviso that it is bonded to the rest of the molecule of formula (II) via a ring carbon atom. Preferably, a heterocyclic radical $R_1$ or $R_2$ is lower alkyl-piperazinyl or especially preferred tetrahydropyranyl. If one of the two radicals $R_1$ and $R_2$ represents a heterocyclic radical, the other is preferably hydrogen.

A heterocyclic radical $R_3$ of formula (II) is as defined above for a heterocyclic radical with the proviso that it is bonded to Q via a ring carbon atom if X is not present. Preferably, a heterocyclic radical $R_3$ is benzodioxolyl, pyridyl substituted by hydroxy or lower alkoxy, or especially preferred indolyl substituted by halogen and lower alkyl. If $R_3$ is pyridyl substituted by hydroxy then the hydroxy group is preferably attached to a ring carbon atom adjacent to the ring nitrogen atom.

A heterocyclic radical $R_4$ of formula (II) is as defined above for a heterocyclic radical and is preferably pyrrolidinyl, piperidyl, lower alkyl-piperazinyl, morpholinyl or pyridyl.

If $R_1$ and $R_2$ of formula (II), together with the nitrogen atom to which they are attached, form a heterocyclic radical, the heterocyclic radical is as defined above for a heterocyclic radical and represents preferably pyrrolidinyl, piperidyl, lower alkyl-piperazinyl, di-lower alkyl-piperazinyl or morpholinyl.

An unsubstituted or substituted aromatic radical $R_3$ of formula (II) has up to 20 carbon atoms and is unsubstituted or substituted, e.g., in each case unsubstituted or substituted phenyl. Preferably, an unsubstituted aromatic radical $R_3$ is phenyl. A substituted aromatic radical $R_3$ is preferably phenyl substituted by one or more substituents selected independently of one another from the group consisting of unsubstituted or substituted lower alkyl, amino, *N*-lower alkylamino, *N*,*N*-di-lower alkylamino, *N*-lower alkanoylamino, *N*,*N*-di-lower alkanoylamino, hydroxy, lower alkoxy, lower alkanoyl, lower alkanoyloxy, cyano, nitro, carboxy, lower alkoxycarbonyl, carbamoyl, *N*-lower alkyl-carbamoyl, *N*,*N*-di-lower alkyl-carmoyl, amidino, guanidino, ureido, mercapto, lower alkylthio and halogen. Most preferably, a substituted aromatic radical $R_3$ is phenyl substituted by one or more radicals selected independently of one another from the group consisting

of lower alkyl, amino, hydroxy, lower alkoxy, halogen and benzyloxy.

Halogen of formula (II) is primarily fluoro, chloro, bromo or iodo, especially fluoro, chloro or bromo.

$C_1$-$C_7$Alkylene of formula (II) may be branched or unbranched and is, in particular, $C_1$-$C_3$alkylene.

$C_1$-$C_7$Alkylene G of formula (II) is preferably $C_1$-$C_3$alkylene, most preferably methylene (-$CH_2$-).

If G of formula (II) is not $C_1$-$C_7$alkylene, it preferably represents -C(=O)-.

$C_1$-$C_7$Alkylene X of formula (II) is preferably $C_1$-$C_3$alkylene, most preferably methylene (-$CH_2$-) or ethan-1,1-diyl (-CH($CH_3$)-).

Q of formula (II) is preferably -NH-.

Z of formula (II) is preferably oxygen or sulfur, most preferably oxygen.

In one embodiment, a particularly preferred protein tyrosine kinase inhibitor for use in the invention is {6-[4-(4-ethyl-oiperazin-1-ylmethyl)-phenyl]-7*H*-pyrrolo[2,3-*d*]pyrimidin-4-yl}-(1-[phenyl-ethyl)-amine or a pharmaceutically acceptable salt thereof.

Rapamycin is a known macrolide antibiotic produced by *Streptomyces hygroscopicus*. Suitable derivatives of rapamycin of the present invention include, e.g., compounds of formula (III)

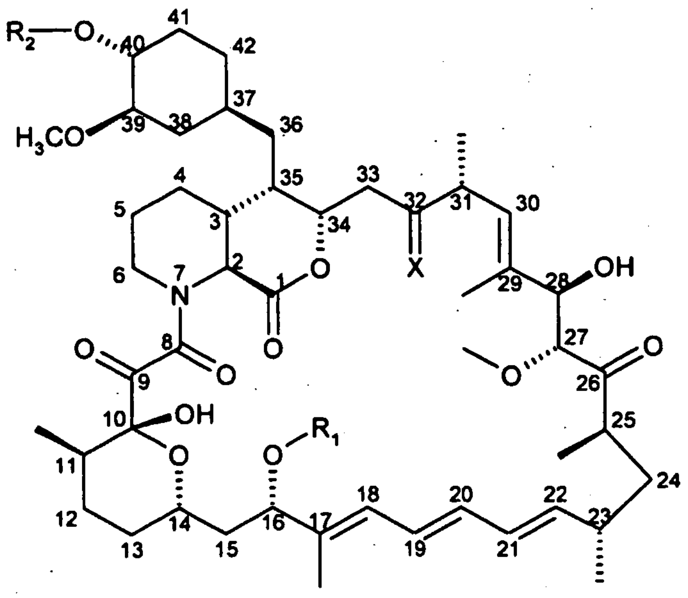

**(III)**

,

wherein

$R_1$ is $CH_3$ or $C_3$-$C_6$alkynyl;

$R_2$ is H or -$CH_2$-$CH_2$-OH; and

X is =O, (H,H) or (H,OH),

provided that $R_2$ is other than H when X is =O and $R_1$ is $CH_3$.

Compounds of formula (III) are disclosed, e.g., in WO 94/09010, WO 95/16691 or WO 96/41807, which are incorporated herein by reference. They may be prepared as disclosed or by analogy to the procedures described in these references.

In one embodiment, a particularly preferred derivative of rapamycin of formula (III) is one,

where

$R_1$ is $CH_3$;

$R_2$ is $CH_2$-$CH_2$-OH; and

X is O.

Furthermore, the stricture of the active agents mentioned herein by name may be taken from the actual edition of the standard compendium "The Merck index" or from databases, e.g., Patents International, e.g., IMS World Publications. Any person skilled in the art is fully enabled, based on these references, to manufacture and test the pharmaceutical indications and properties in standard test models, both *in vitro* and *in vivo*.

In view of the close relationship between the novel compounds in free form and in the form of their salts, including those salts that can be used as intermediates, e.g., in the purification or identification of the novel compounds, hereinbefore and hereinafter any reference to the free compounds is to be understood as referring also to the corresponding salts, as appropriate and expedient.

The compounds used as combination partners (a) and (b), and optionlly (c), disclosed herein can be prepared

and administered as described in the cited documents, respectively.

It will be understood that references to the combination partners (a) and (b), and optionally (c), are meant to also include the pharmaceutically acceptable salts. If these combination partners (a) and (b), and optionally (c), have, e.g., at least one basic center, they can form acid addition salts. Corresponding acid addition salts can also be formes having, if desired, an additionally present basic center. The combination partners (a) and (b), and optionally (c), having an acid group, e.g., COOH, can also form salts with bases. The combination partner (a) and (b), and optionally (c), or a pharmaceutically acceptable salt thereof may also be used in form of a hydrate or include other solvents used for crystallization.

A combination which comprises:

(a) an epothilone derivative of formula (I),
in which compound
A represents O or $NR_N$, wherein $R_N$ is hydrogen or lower alkyl;
R is hydrogen or lower alkyl; and
Z is O or a bond; and

(b) a protein tyrosine kinase inhibitor of formula (II), preferably {6-[4-(4-ethyl-piperazin-1-ylmethyl)-phenyl]-7*H*-pyrrolo [2,3-*d*]pyrimidin-4-yl}-(1-[phenyl-ethyl)-amine and optionally (a) a derivative of rapamycin as defined in formula (III), preferably

where
$R_1$ is $CH_3$;
$R_2$ is $-CH_2-CH_2-OH$; and
X is O,

in which the active ingredients are present in each case in free form or in the form of a pharmaceutically acceptable salt and optionally at least one pharmaceutically acceptable carrier, will be referred to hereinafter as a COMBINATION OF THE INVENTION.

The COMBINATIONS OF THE INVENTION inhibits the growth of solid tumors, but also liquid tumors. The nature of proliferative diseases like solid tumor diseases is multifactorial. Under certain circumstances, drugs with different mechanisms of action may be combined. However, just considering any combination of drugs having different mode of action does not necessarily lead to combinations with advantageous effects.

It is one objective of this invention to provide a pharmaceutical composition comprising a quantity, which is jointly therapeutically effective against a proliferative disease comprising the COMBINATION OF THE INVENTION. In this composition, the combination partners (a) and (b), and optionally (c), can be administered together, one after the other or separately in one combined unit dosage form or in two separate unit dosage forms. The unit dosage form may also be a fixed combination.

The pharmaceutical compositions according to the invention can be prepared in a manner known per se and are those suitable for enteral, such as oral or rectal; and parenteral administration to mammals (warm-blooded animals), including man, comprising a therapeutically effective amount of at least one pharmacologically active combination partner alone or in combination with one or more pharmaceutically acceptable carries, especially suitable for enteral or parenteral application.

The novel pharmaceutical composition contain, e.g., from about 10% to about 100%, preferably from about 20% to about 60%, of the active ingredients. Pharmaceutical preparations for the combination therapy for enteral or parenteral administration are, e.g., those in unit dosage forms, such as sugar-coated tablets, tablets, capsules or suppositories, and furthermore ampoules. If not indicated otherwise, these are prepared in a manner known per se, e.g., by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units.

In preparing the compositions for oral dosage form, any of the usual pharmaceutical media may be employed, such as, e.g., water, glycols, oils, alcohols, flavoring agents, preservatives, coloring agents; or carriers, such as starches, sugars, microcrystalline cellulose, diluents, granulating agents, lubricants, binders, disintegrating agents and the like in the case of oral solid preparations, such as, e.g., powders, capsules and tablets, with the solid oral preparations being preferred over the liquid preparations. Because of their ease of administration, tablets and capsules represent the most advantageous oral dosage unit form in which case solid pharmaceutical carriers are obviously employed.

In particular, a therapeutically effective amount of each of the combination partners of the COMBINATION OF THE INVENTION may be administered simultaneously or sequentially and in any order, and the components may be administered separately or as a fixed combination. For example, the method of delay of progression or treatment of a

proliferative disease according to the invention may comprise:

(a) administration of the first combination partner in free or pharmaceutically acceptable salt form; and
(b) administration of the second combination partner in free or pharmaceutically acceptable salt form; and optionally
(c) administration of the third combination partner in free or pharmaceutically acceptable salt form, simultaneously or sequentially in any order, in jointly therapeutically effective amounts, preferably in synergistically effective amounts, e.g., in daily dosages corresponding to the amounts described herein.

The individual combination partners of the COMBINATION OF THE INVENTION can be administered separately at different times during the course of therapy or concurrently in divided or single combination forms. Furthermore, the term administering also encompasses the use of a pro-drug of a combination partner that convert *in vivo* to the combination partner as such. The instant invention is therefore to be understood as embracing all such regimes of simultaneous or alternating treatment and the term "administering" is to be interpreted accordingly.

The effective dosage of each of the combination partners employed in the COMBINATION OF THE INVENTION may vary depending on the particular compound or pharmaceutical composition employed, the mode of administration, the condition being treated and the severity of the condition being treated. Thus, the dosage regimen the COMBINATION OF THE INVENTION is selected in accordance with a variety of factors including the route of administration and the renal and hepatic function of the patient. A physician, clinician or veterinarian of ordinary skill can readily determine and prescribe the effective amount of the single active ingredients required to prevent, counter or arrest the progress of the condition. Optimal precision in achieving concentration of the active ingredients within the range that yields efficacy without toxicity requires a regimen based on the kinetics of the active ingredients' availability to target sites. This involves a consideration of the distribution, equilibrium, and elimination of the active ingredients.

If the warm-blooded animal is a human, the dosage of a compound of formula (I') or (I) is preferably in the range of about 0.25-75 mg/$m^2$, preferably 0.5-50 mg/$m^2$, e.g., 2.5, mg/$m^2$ once weekly for 2-4 weeks, e.g., 3 weeks, followed by 6-8 days off in the case of an adult patient. Epothilone B is preferably administered in a dose which is calculated according to the formula (IV)

$$\text{single dose (mg/m}^2\text{)} = (0.1 \text{ to } y) \times N \qquad \text{(IV)}$$

wherein
N is the number of weeks between treatments; and
y is 6,
wherein epothilone B is administered in more than one treatment cycle after an interval of 1-6 weeks after the preceding treatment.

In one preferred embodiment of the invention, epothilone B is administered weekly in a dose that is between about 0.1-6 mg/$m^2$, preferably between 0.1 and 3 mg/$m^2$, e.g., 2.5 or 3.0 mg/$m^2$, for 3 weeks after an interval of 1-6 weeks, especially an interval of 1 week, after the preceding treatment. In another embodiment of the invention said epothilone B is preferably administered to a human every 18-24 days in a dose that is between about 0.3 and 12 mg/$m^2$.

The protein tyrosine kinase inhibitors of formula (II) of the present invention can be administered for an individual having a bodyweight of about 70 kg the daily dose from approximately 0.1 g to approximately 5 g, preferably from approximately 0.5 g to approximately 2 g, of a compound of the present invention.

In general, results are achieved on administration of a derivative rapamycin of formula (II) of the present invention at daily dosage rates of the order of about 0.1-25 mg as a single dose or in divided doses. Suitable unit dosage forms for derivatives of rapamycin of the present invention for oral administration comprise from ca. 0.05-10 mg active ingredient.

In a preferred embodiment of the invention, the COMBINATION OF THE INVENTION comprises a protein tyrosine kinase inhibitor which is {6-[4-(4-ethyl-piperazin-1-ylmethyl)-phenyl]-7*H*-pyrrolo[2,3-*d*]pyrimidin-4-yl}-(1-[phenyl-ethyl)-amine or a pharmaceutically acceptable salt thereof.

In another embodiment of the invention, the COMBINATION OF THE INVENTION comprises a derivative of rapamycin of formula (III),
wherein
$R_1$ is $CH_3$;
$R_2$ is $-CH_2-CH_2-OH$; and
X is O.

The COMBINATION OF THE INVENTION can be a combined preparation or a pharmaceutical composition.

Furthermore, the present invention pertains to the use of a COMBINATION OF THE INVENTION for the delay

of progression or treatment of a proliferative disease and for the preparation of a medicament for the delay of progression or treatment of a proliferative disease.

Additionally, the present invention pertains to the use of epothilone of formula (I') or (I) in combination with a protein tyrosine kinase inhibitor of formula (II) and optionally a derivative of rapamycin of formula (III) for the preparation of a medicament for the delay of progression or treatment of a proliferative disease.

Moreover, the present invention provides a commercial package comprising as active ingredients COMBINATION OF THE INVENTION, together with instructions for simultaneous, separate or sequential use thereof in the delay of progression or treatment of a proliferative disease.

## Claims

1. A combination which comprises:

   (a) an epothilone derivative of formula (I')

   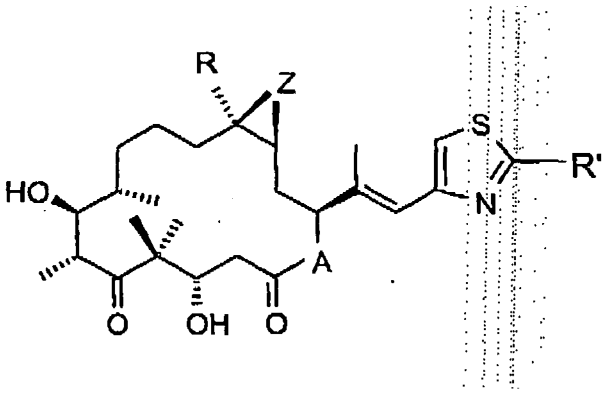

   (I')

   wherein A represents O or $NR_N$, wherein $R_N$ is hydrogen or lower alkyl, R is hydrogen or lower alkyl including not more than 7 carbon atoms, R' is methyl, and Z is O or a bond, in free form or in the form of a pharmaceutically acceptable salt; and

   (b) a protein tyrosine kinase inhibitor {6-[4-(4-ethyl-piperazin-1-ylmethyl)-phenyl]-7*H*-pyrrolo[2,3-*d*]pyrimidin-4-yl]-(1-[phenyl-ethyl)-amine

   And , in which the active ingredients (a) and (b) are present in each case in free form or in the form of a pharmaceutically acceptable salt and optionally at least one pharmaceutically acceptable carrier; for simultaneous, separate or sequential use.

2. The combination as claimed in Claim 1, further comprises a rapamycin derivative in free form or in the form of a pharmaceutically acceptable salt.

3. The combination as claimed in Claim 2 wherein the rapamycin derivative is a compound of formula (III)

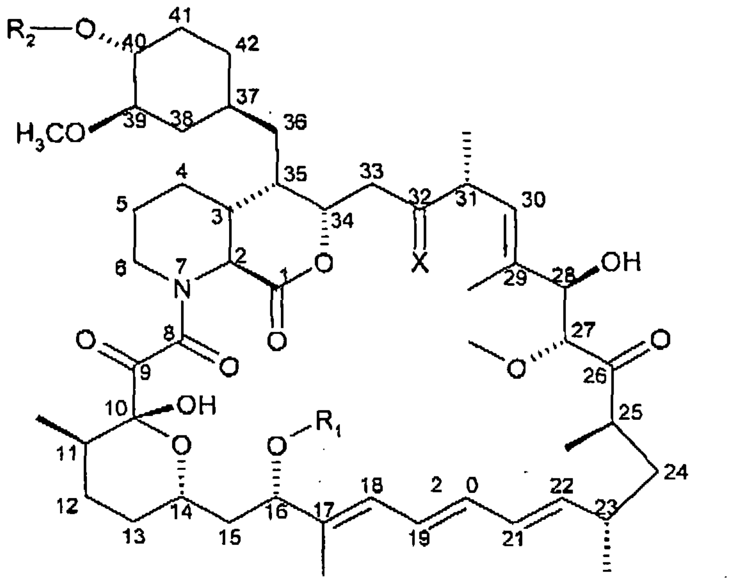

(III)

wherein
$R_1$ is $CH_3$ or $C_3$-$C_6$alkynyl;
$R_2$ is H or $-CH_2-CH_2-OH$; and
X is =O, (H,H) or (H,OH), provided that $R_2$ is other than H when X is =O and $R_1$ is $CH_3$; and a pharmaceutically acceptable salt thereof.

**4.** The combination as claimed in Claim 3,
wherein
$R_1$ is $CH_3$;
$R_2$ is $-CH_2-CH_2-OH$; and
X is O.

**5.** The combination as claimed in any one of Claims 1-4, comprising an epothilone derivative of formula (I),

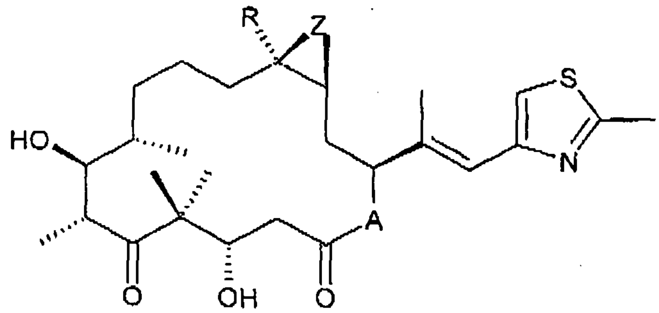

(I)

wherein
A represents O;
R is lower alkyl including not more than 7 carbon atoms or hydrogen; and
Z is O or a bond.

**6.** The combination as claimed in claim 5, wherein R is methyl and Z is O.

**7.** The combination as claimed in any one of Claims 1-6, which is a combined preparation or a pharmaceutical composition.

**8.** A pharmaceutical composition comprising a quantity which is jointly therapeutically effective against a proliferative

disease of a pharmaceutical combination as claimed in any one of Claims 1-7 and at least one pharmaceutically acceptable carrier.

**9.** The combination as claimed in any one of Claims 1-7, for use in the delay of progression or treatment of a proliferative disease.

**10.** Use of a combination as claimed in any one of Claims 1-7, for the preparation of a medicament for the treatment of a proliferative disease.

**11.** A commercial package comprising:

(a) an epothilone derivative of formula (I)

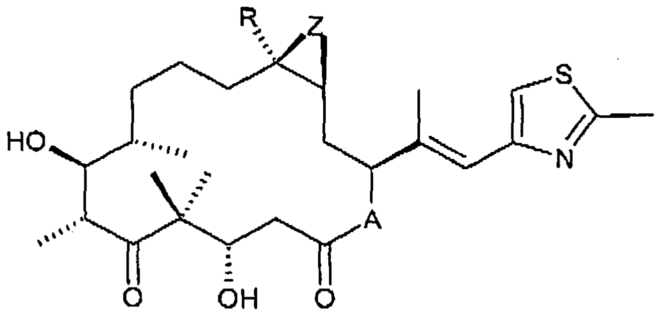

**(I)**

,

wherein
A represents O or $NR_N$, wherein $R_N$ is hydrogen or lower alkyl including not more than 7 carbon atoms;
R is hydrogen or lower alkyl; and
Z is O or a bond; and
(b) a protein tyrosine kinase inhibitor {6-[4-(4-ethyl-piperazin-1-ylmethyl)-phenyl]-7*H*-pyrrolo[2,3-*d*]pyrimidin-4-yl}-(1-[phenyl-ethyl)-amine or a pharmaceutically acceptable salt thereof
together with instructions for simultaneous, separate or sequential use thereof in the delay of progression or treatment of a proliferative disease.

**12.** The commercial package as claimed in Claim 11, further comprising a rapamycin derivative.

**13.** The commercial package as claimed in Claim 12, wherein the rapamycin derivative is a compound of formula (III)

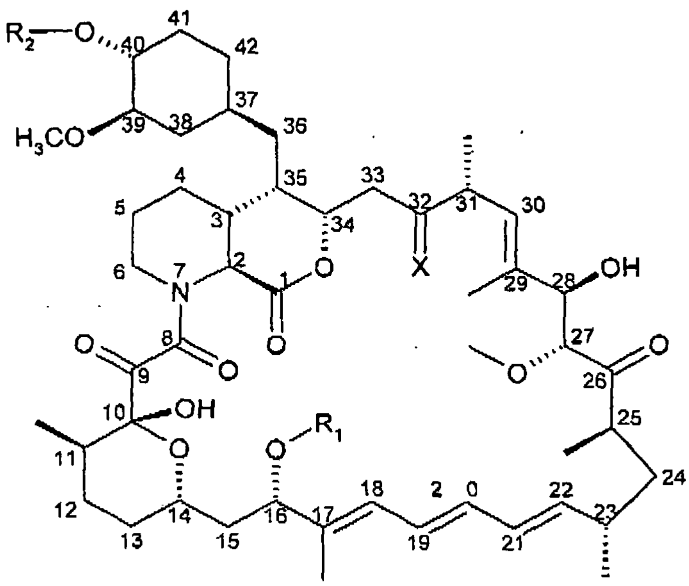

(III)

wherein
$R_1$ is $CH_3$ or $C_3$-$C_6$alkynyl;
$R_2$ is H or $-CH_2-CH_2-OH$; and
X is =O, (H,H) or (H,OH), provided that $R_2$ is other than H when X is =O and $R_1$ is $CH_3$; and a pharmaceutically acceptable salt thereof.

**14.** The commercial package as claimed in Claim 13,
wherein
$R_1$ is $CH_3$;
$R_2$ is $-CH_2-CH_2-OH$; and
X is O.

**15.** The commercial package as claimed in any one of Claims 11-14, comprising an epothilone derivative of formula (I),

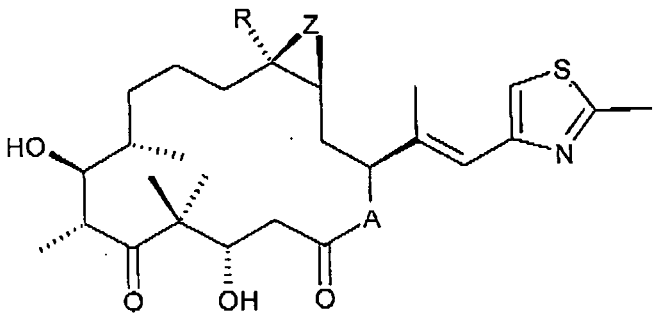

(I)

wherein
A represents O;
R is lower alkyl including not more than 7 carbon atoms or hydrogen; and
Z is O or a bond.

**16.** The commercial package as claimed in claim 15, wherein R is methyl and Z is O.

**Patentansprüche**

1. Kombination, die die folgenden Bestandteile umfasst:

   (a) ein Epothilonderivat der Formel (I')

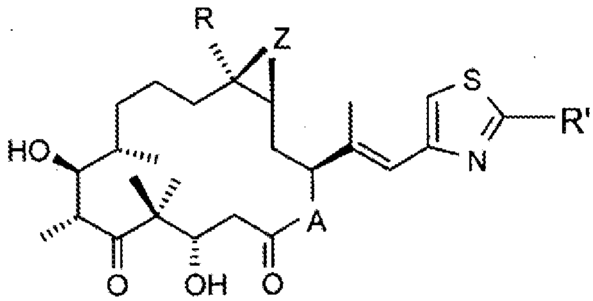

(I')

   worin A für O oder $NR_N$ steht, worin $R_N$ für Wasserstoff oder Niedrigalkyl steht, R für Wasserstoff oder Niedrigalkyl, einschließlich von nicht mehr als 7 Kohlenstoffatomen, steht, R' für Methyl steht und Z für O oder eine Bindung steht, in freier Form oder in Form eines pharmazeutisch akzeptablen Salzes und
   (b) den Proteintyrosinkinaseinhibitor {6-[4-(4-Ethylpiperazin-1-ylmethyl)-phenyl]-7H-pyrrolo[2,3-d]pyrimidin-4-yl}-(1-phenyl-ethyl)-amin,

   wobei die wirksamen Bestandteile (a) und (b) in jedem Fall in freier Form oder in Form eines pharmazeutisch akzeptablen Salzes vorhanden sind, und optional mindestens einen pharmazeutisch akzeptablen Träger, zur gleichzeitigen, getrennten oder aufeinanderfolgenden Verwendung.

2. Kombination nach Anspruch 1, die ferner ein Rapamycinderivat in freier Form oder in Form eines pharmazeutisch akzeptablen Salzes umfasst.

3. Kombination nach Anspruch 2, wobei das Rapamycinderivat eine Verbindung der Formel (III)

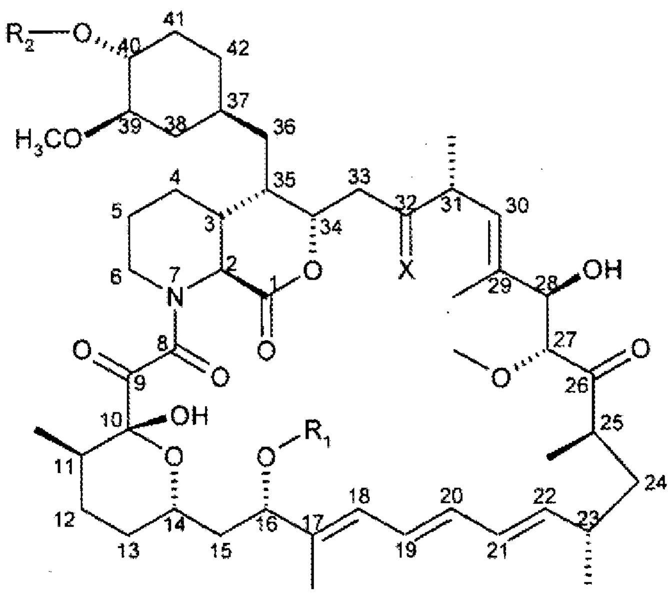

**(III)**

   worin
   $R_1$ für $CH_3$ oder $C_3$-$C_6$-Alkinyl steht,

$R_2$ für H oder $-CH_2-CH_2-OH$ steht und
X für =O, (H,H) oder (H,OH) steht,
wobei gilt, dass $R_2$ von H verschieden ist, wenn X für =O steht und $R_1$ für $CH_3$ steht,
und ein pharmazeutisch akzeptables Salz hiervon ist.

**4.** Kombination nach Anspruch 3,
worin
$R_1$ für $CH_3$ steht,
$R_2$ für $-CH_2-CH_2-OH$ steht und
X für O steht.

**5.** Kombination nach einem der Ansprüche 1 bis 4, die ein Epothilonderivat der Formel (I) umfasst

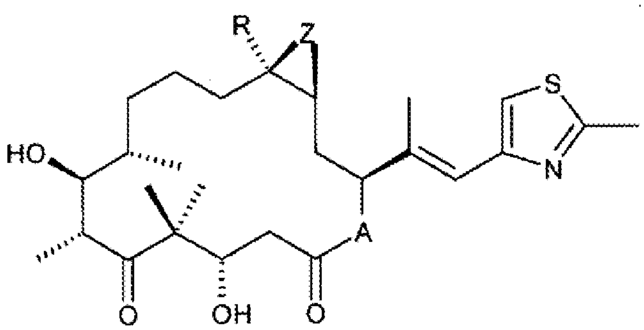

**(I)**

worin
A für O steht,
R für Niedrigalkyl, einschließlich von nicht mehr als 7 Kohlenstoffatomen, oder für Wasserstoff steht und
Z für O oder eine Bindung steht.

**6.** Kombination nach Anspruch 5, worin R für Methyl steht und Z für O steht.

**7.** Kombination nach einem der Ansprüche 1 bis 6, bei der es sich um eine Kombinationszubereitung oder eine pharmazeutische Zusammensetzung handelt.

**8.** Pharmazeutische Zusammensetzung, die eine Menge einer pharmazeutischen Kombination gemäß einem der Ansprüche 1 bis 7, die gegenüber einer proliferativen Erkrankung gemeinsam therapeutisch wirksam ist, und mindestens einen pharmazeutisch akzeptablen Träger umfasst.

**9.** Kombination nach einem der Ansprüche 1 bis 7 zur Verwendung bei der Verzögerung der Progression oder Behandlung einer proliferativen Erkrankung.

**10.** Verwendung einer Kombination nach einem der Ansprüche 1 bis 7 zur Herstellung eines Medikaments zur Behandlung einer proliferativen Erkrankung.

**11.** Kommerzielle Packung, die die folgenden Bestandteile umfasst:

(a) ein Epothilonderivat der Formel (I)

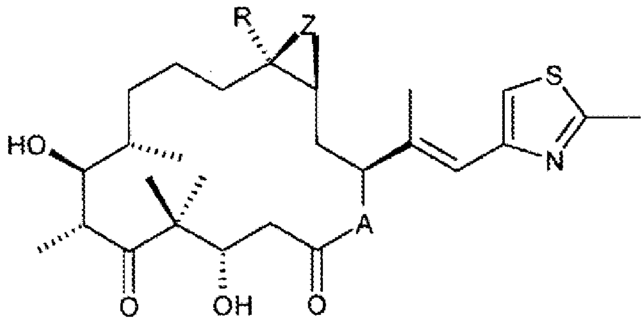

(I)

worin
A für O oder $NR_N$ steht, worin $R_N$ für Wasserstoff oder Niedrigalkyl, einschließlich von nicht mehr al 7 Kohlenstoffatomen, steht,
R für Wasserstoff oder Niedrigalkyl steht und
Z für O oder eine Bindung steht und
(b) den Proteintyrosinkinaseinhibitor {6-[4-(4-Ethylpiperazin-1-ylmethyl)-phenyl]-7H-pyrrolo-[2,3-d]pyrimidin-4-yl}-(1-phenyl-ethyl)-amin oder ein pharmazeutisch akzeptables Salz hiervon zusammen mit Instruktionen zur simultanen, getrennten oder aufeinanderfolgenden Verwendung hiervon bei der Verzögerung der Progression oder Behandlung einer proliferativen Erkrankung.

**12.** Kommerzielle Packung nach Anspruch 11, die ferner ein Rapamycinderivat umfasst.

**13.** Kommerzielle Packung nach Anspruch 12, wobei das Rapamyinderivat eine Verbindung der Formel (III)

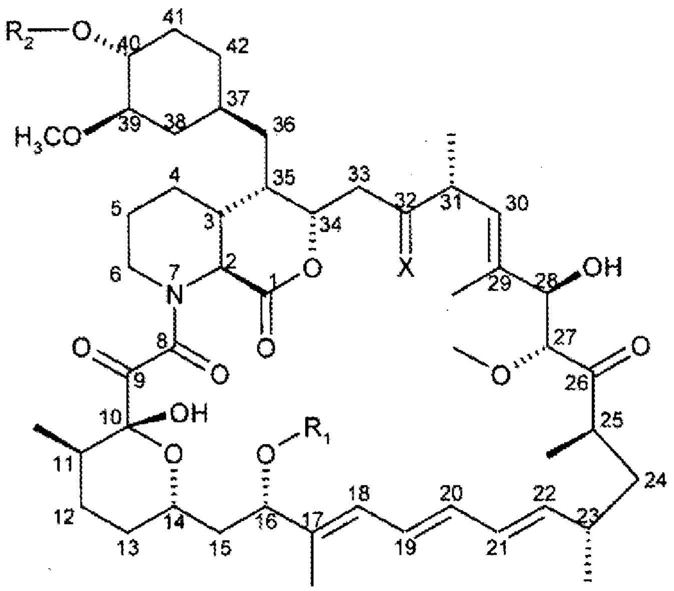

(III)

worin
$R_1$ für $CH_3$ oder $C_3$-$C_6$-Alkinyl steht,
$R_2$ für H oder $-CH_2$-$CH_2$-OH steht und
X für =O, (H,H) oder (H,OH) steht,
wobei gilt, dass $R_2$ von H verschieden ist, wenn X für =O steht und $R_1$ für $CH_3$ steht,
und ein pharmazeutisch akzeptables Salz hiervon ist.

**14.** Kommerzielle Packung nach Anspruch 13,
worin

$R_1$ für $CH_3$ steht,
$R_2$ für $-CH_2-CH_2-OH$ steht und
X für O steht.

**15.** Kommerzielle Packung nach einem der Ansprüche 11 bis 14, umfassend ein Epothilonderivat der Formel (I)

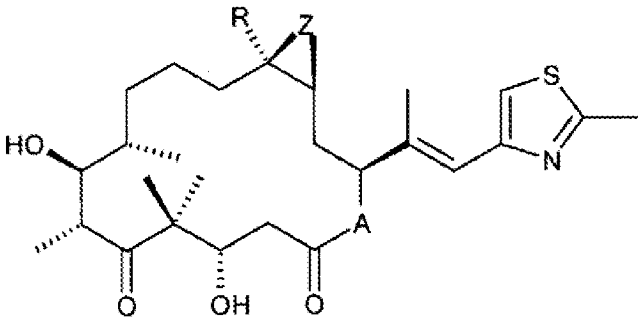

(I)

worin
A für O steht,
R für Niedrigalkyl, einschließlich von nicht mehr als 7 Kohlenstoffatomen, oder für Wasserstoff steht und
Z für O oder eine Bindung steht.

**16.** Kommerzielle Packung nach Anspruch 15, wobei R für Methyl steht und Z für O steht.

**Revendications**

**1.** Composition comprenant :

(a) un dérivé d'épothilone de formule (I')

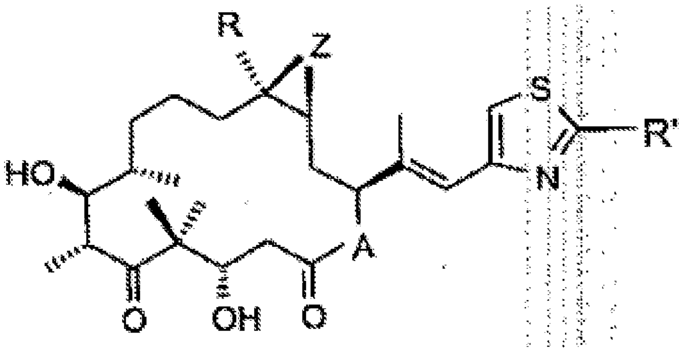

(I')

dans laquelle A représente O ou $NR_N$, où $R_N$ est l'hydrogène ou un alkyle inférieur, R est l'hydrogène ou un alkyle inférieur ne comprenant pas plus de 7 atomes de carbone, R' est le méthyle, et Z est O ou une liaison, sous forme libre ou sous forme de sel pharmaceutiquement acceptable ; et
(b) un inhibiteur de la protéine tyrosine kinase {6-[4-(4-éthyl-pipérazin-1-ylméthyl)-phényl]-7*H*-pyrrolo[2,3-*d*]pyrimidin-4-yl}-(1-phényl-éthyl)-amine

et dans laquelle les principes actifs (a) et (b) sont présents dans chaque cas sous forme libre ou sous forme de sel pharmaceutiquement acceptable et facultativement au moins un véhicule pharmaceutiquement acceptable ; pour une utilisation simultanée, séparée ou séquentielle.

**2.** Combinaison selon la revendication 1, comprenant en outre un dérivé de rapamycine sous forme libre ou sous forme de sel pharmaceutiquement acceptable.

**3.** Combinaison selon la revendication 2, dans laquelle le dérivé de rapamycine est un composé de formule (III)

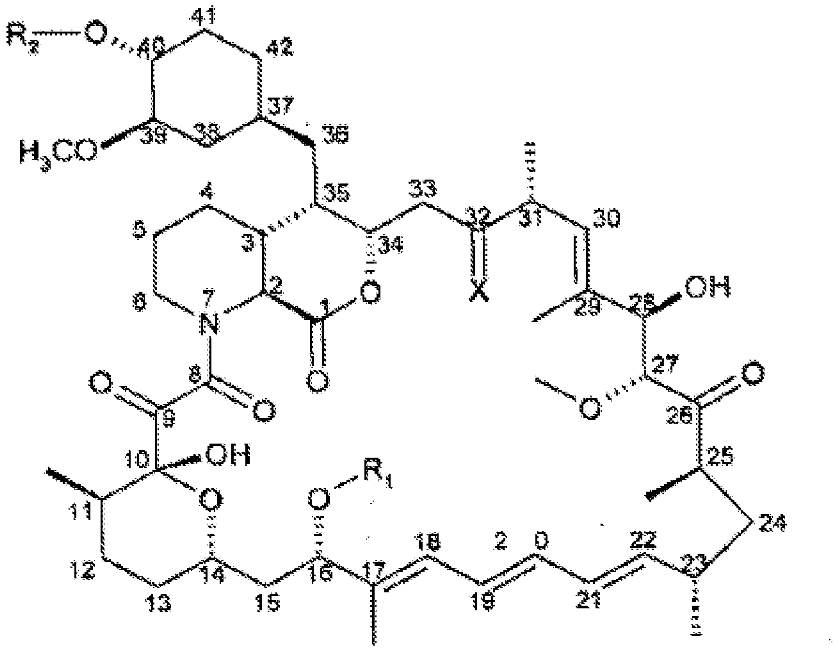

(III)

dans laquelle
$R_1$ est $CH_3$ ou un alcynyle en $C_3$-$C_6$ ;
$R_2$ est H ou $-CH_2-CH_2-OH$ ; et
X est =O, (H,H) ou (H,OH), à la condition que $R_2$ ne soit pas H lorsque X est =O et $R_1$ est $CH_3$ ;
et un sel pharmaceutiquement acceptable de celle-ci.

**4.** Combinaison selon la revendication 3, dans laquelle
$R_1$ est $CH_3$ ;
$R_2$ est $-CH_2-CH_2-OH$ ; et
X est O.

**5.** Combinaison selon l'une quelconque des revendications 1 à 4, comprenant un dérivé d'épothilone de formule (I),

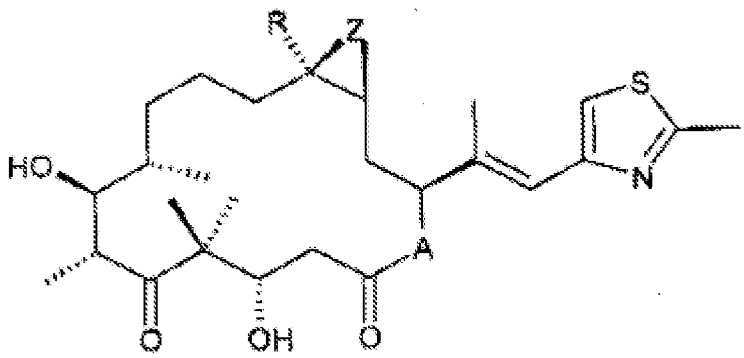

(I)

dans laquelle
A représente O ;

R est un alkyle inférieur ne comprenant pas plus de 7 atomes de carbone ; et
Z est O ou une liaison.

**6.** Combinaison selon la revendication 5, dans laquelle R est le méthyle et Z est O.

**7.** Combinaison selon l'une quelconque des revendications 1 à 6, laquelle est une préparation combinée ou une composition pharmaceutique.

**8.** Composition pharmaceutique comprenant une quantité qui est conjointement thérapeutiquement efficace contre une maladie proliférative d'une combinaison pharmaceutique selon l'une quelconque des revendications 1 à 7 et au moins un véhicule pharmaceutiquement acceptable.

**9.** Combinaison selon l'une quelconque des revendications 1 à 7, pour une utilisation dans le retardement de l'évolution ou le traitement d'une maladie proliférative.

**10.** Utilisation d'une combinaison selon l'une quelconque des revendications 1 à 7 pour la préparation d'un médicament destiné au traitement d'une maladie proliférative.

**11.** Conditionnement commercial comprenant :

(a) un dérivé d'épothilone de formule (I)

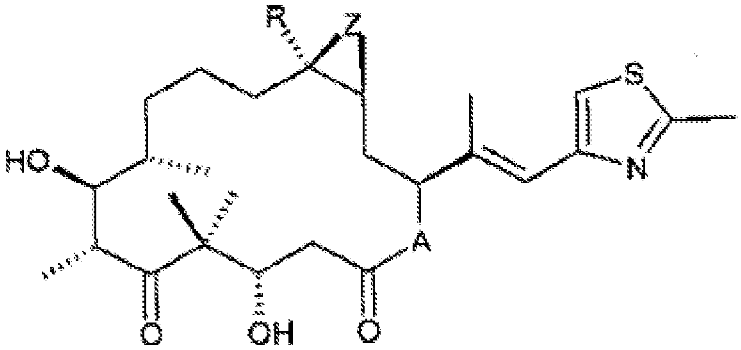

**(I)**

dans laquelle
A représente O ou $NR_N$, où $R_N$ est l'hydrogène ou un alkyle inférieur ne comprenant pas plus de 7 atomes de carbone,
R est l'hydrogène ou un alkyle inférieur ; et
Z est O ou une liaison ; et
(b) un inhibiteur de la protéine tyrosine kinase {6-[4-(4-éthyl-pipérazin-1-ylméthyl)-phényl]-7*H*-pyrrolo[2,3-*d*] pyrimidin-4-yl}-(1-phényl-éthyl)-amine ou un sel pharmaceutiquement acceptable de celui-ci

conjointement avec des instructions pour son utilisation simultanée, séparée ou séquentielle dans le retardement de l'évolution ou le traitement d'une maladie proliférative.

**12.** Conditionnement commercial selon la revendication 11, comprenant en outre un dérivé de rapamycine.

**13.** Conditionnement commercial selon la revendication 12, dans lequel le dérivé de rapamycine est un composé de formule (III)

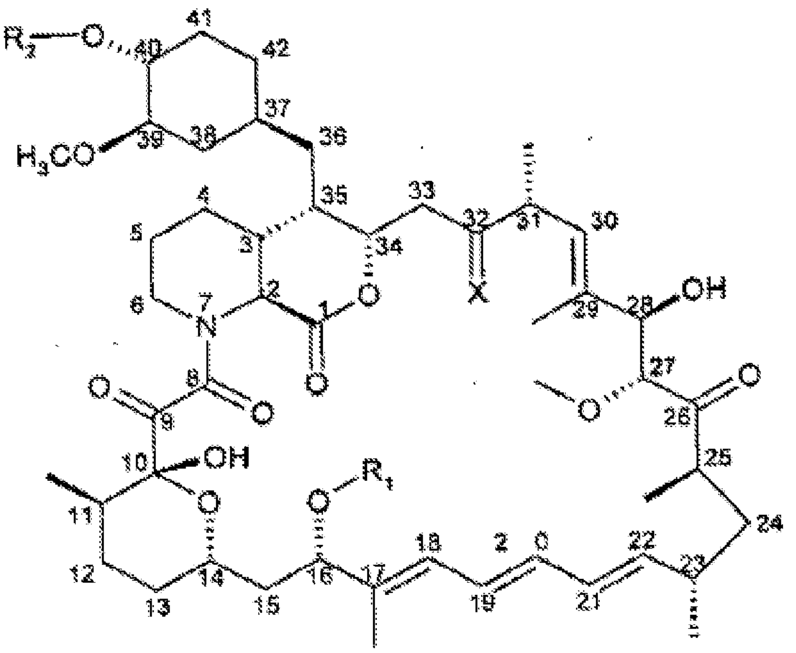

(III)

dans laquelle
$R_1$ est $CH_3$ ou un alcynyle en $C_3$-$C_6$ ;
$R_2$ est H ou $-CH_2-CH_2-OH$ ; et
X est =O, (H,H) ou (H,OH), à la condition que $R_2$ ne soit pas H lorsque X est =O et $R_1$ est $CH_3$ ;
et un sel pharmaceutiquement acceptable de celui-ci.

**14.** Conditionnement commercial selon la revendication 13, dans lequel
$R_1$ est $CH_3$ ;
$R_2$ est $-CH_2-CH_2-OH$ ; et
X est O.

**15.** Conditionnement commercial selon l'une quelconque des revendications 11 à 14, comprenant un dérivé d'épothilone de formule (I),

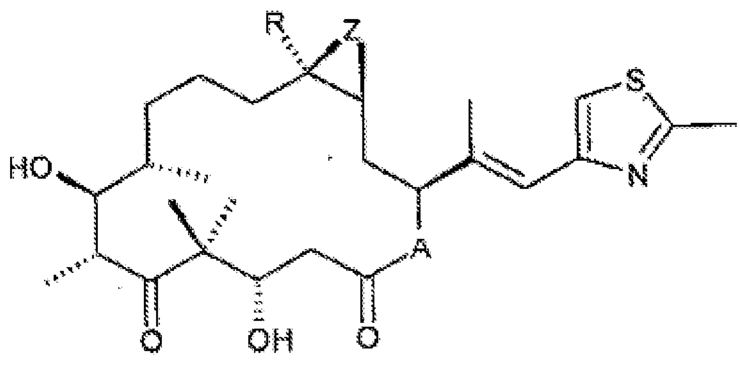

(I)

dans laquelle
A représente O ;
R est un alkyle inférieur ne comprenant pas plus de 7 atomes de carbone ; et
Z est O ou une liaison.

**16.** Conditionnement commercial selon la revendication 15, dans lequel R est le méthyle et Z est O.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 4138042, Hoefle
- WO 9310121 A
- US 6194181 B
- WO 9825929 A
- WO 9808849 A
- WO 9943653 A
- WO 0031247 A
- WO 9939694 A
- WO 03013541 A
- WO 9409010 A
- WO 9516691 A
- WO 9641807 A


### Non-patent literature cited in the description


- **Gerth et al.** *J Antibiot,* 1966, vol. 49, 560-563